(19) Europäisches Patentamt
European Patent Office
Office européen des brevets

(11) Publication number: 0 526 520 B1

# (12) EUROPEAN PATENT SPECIFICATION

(45) Date of publication of patent specification: 30.11.94

(51) Int. Cl.5: A61K 7/48, C11D 3/37

(21) Application number: 91908306.3

(22) Date of filing: 09.04.91

(86) International application number:
PCT/US91/02443

(87) International publication number:
WO 91/16037 (31.10.91 91/25)

## (54) MILDNESS ADDITIVES FOR SKIN CLEANSING COMPOSITIONS.

(30) Priority: 16.04.90 US 509461

(43) Date of publication of application:
10.02.93 Bulletin 93/06

(45) Publication of the grant of the patent:
30.11.94 Bulletin 94/48

(84) Designated Contracting States:
DE ES FR GB IT SE

(56) References cited:
FR-A- 2 398 496
GB-A- 2 144 133
US-A- 4 552 755

(73) Proprietor: MINNESOTA MINING AND MANUFACTURING COMPANY
3M Center,
P.O. Box 33427
St. Paul, Minnesota 55133-3427 (US)

(72) Inventor: RANDEN, Neil A.
Post Office Box 33427
Saint Paul, MN 55133-3427 (US)
Inventor: CHANG, Robert W.H.
Post Office Box 33427
Saint Paul, MN 55133-3427 (US)

(74) Representative: Baillie, Iain Cameron
c/o Ladas & Parry
Altheimer Eck 2
D-80331 München (DE)

EP 0 526 520 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The invention relates to mildness additives for skin cleansing compositions. More specifically, it relates to polymeric mildness additives for skin cleansing bars and liquids.

### Background of the Invention

Since early time, people have combined fats and potash to create compositions that would be useful as a cleanser, especially for the skin. Over the years variations and improvements have been made to provide a wide selection of cleansing products with different properties.

U.S. Patent No. 2,820,768 to Fromont discloses transparent toilet soaps in a solid form consisting of (1) a transparent sodium soap prepared by saponification of a mixture of tallow, coconut oil and castor oil, (2) a triethanolammonium salt of stearic acid, and (3) a quantity of triethanolamine in excess over the theoretical amount required to fully neutralize the fatty acid and amounting to no less than one mole and no more than two moles per mole of soap forming fatty acid. The toilet bar is described as being both transparent and hard.

U.S. Patent No. 3,406,238 to Freyermuth et al. discloses toiletry and cosmetic compositions comprising as an essential component from 0.1 to about 50% by weight of an alkylated polymer of an N-vinyl heterocyclic monomer. At least 25% on average of the N-vinyl heterocyclic monomer units in the polymer are alkylated with an alkyl group of from 10 to 42 carbon atoms. The presence of this additive in these preparations is disclosed to impart a much smoother texture to the composition and a softening and soothing effect of the composition when applied to the skin.

U.S. Patent No. 3,503,888 to Miller et al. discloses a synthetic detergent toilet bar in which an anionic type detergent is a major component and which has a soapy or slippery feel, good sudsing and rinsability characteristics, and leaves the skin feeling soft and smooth with no tacky sensation. The synthetic detergent toilet bar thus disclosed contains three essential components: a fatty acid ester of a sulfonated phenol, a salt of the fatty acid, and a salt of the sulfonated phenol.

U.S. Patent No. 4,673,525 to Small et al. discloses mild skin cleansers comprising synthetic surfactants, moisturizers, polymeric "skin feel" and mildness aids and soap. The polymeric "skin feel" and mildness aids are fully discussed at col. 7, line 43 to col. 8, line 49. These aids are disclosed to be the cationic, anionic, amphoteric, and the nonionic polymers used in the cosmetic field. The nonionic polymers to be used are disclosed at col. 8, line 11-18, as nonionic polysaccharides and cellulosic nonionic polymers. The disclosure of cationic polymers allows that these polymers may be copolymers with dimethylaminoethylmethacrylate and acrylamide and copolymers of dimethyldiallylammonium chloride and acrylamide, but specifically requires that the ratio of the cationic to neutral monomer units be selected to give a copolymer having a cationic charge.

U.S. Patent No. 4,552,755 to Randen discloses improved oil-in-water moisturizing compositions comprising an oil phase containing at least one emollient oil and an oil soluble acrylate polymer, a water phase and an emulsifying agent. The acrylate polymer is disclosed as having a solubility parameter of 6 to 10 $(cal/cc)^{1/2}$ in poorly hydrogen-bonding solvents. The acrylate polymer increases the substantivity of the moisturizing composition to the skin.

U.S. Patent No. 4,172,122 to Kubik et al. discloses water resistant sunscreening compositions comprising a cosmetically acceptable oil base, at least one water-insoluble ultraviolet light absorbing material which is soluble in the oil base, and a water insoluble acrylate polymer having a solubility parameter of 6 to 10 $(cal/cc)^{1/2}$ in weak hydrogen bonding solvents. The inclusion of the acrylate polymer renders the light absorbent material more water resistant, so that it does not wash away on exposure to water.

Neither reference US-A-4,552,755 nor reference US-A-4,172,122 both of which are mentioned above disclose skin cleaning compositions which have the effect of drying or irritating skin.

Oil based cleansers, such as "Gojo"™ are very effective for cleaning grease and oil based dirt without causing undue irritation to the skin. However, these compositions must themselves be cleaned off in a separate cleaning step to remove the undesirable oily residue left on the skin.

### Summary of the Invention

The present invention is drawn to a skin-cleansing composition comprising a soap composition, a soap/synthetic "combo" composition or a synthetic detergent composition wherein the composition comprises an effective amount of a mildness additive that is a non-water soluble acrylate polymer that is soluble in poorly hydrogen bonding solvents or solvent blends having a solubility parameter of between about 8.4 and 10.4 $(cal/cc)^{1/2}$. The soap composition, soap/synthetic "combo" composition or synthetic detergent composition is a cleansing component having the effect of drying or irritating the skin when it does not contain the mildness additive. The polymer comprises monomers of the formula:

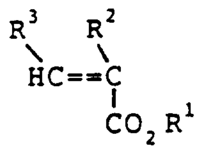

wherein $R^1$ is H or an alkyl radical containing 1 to 18 carbon atoms in cyclic, straight- or branched-chain configuration and is saturated or unsaturated; $R^2$ is hydrogen, lower alkyl or $-CH_2CO_2R^1$; and $R^3$ is hydrogen, methyl or $--CO_2R^1$; provided, that when $R^2$ is not hydrogen, $R^3$ is hydrogen, and when $R^3$ is not hydrogen, $R^2$ is hydrogen.

The incorporation of the indicated polymers reduces the drying and irritating effect of conventional cleansing compositions on the skin.

## Description of Presently Preferred Embodiments

The mildness additives to be incorporated into conventional skin cleansing compositions according to the present invention are acrylate polymers specifically selected to provide the appropriate solubility parameter for the cleansing composition to be used. These acrylate polymers comprise the indicated monomers selected in any manner that will provide the desired solubility parameter for the overall polymer as indicated.

A preferred method of achieving this solubility parameter is by providing certain mole ratio ranges of monomers having identifiable physical property characteristics. In this manner, a suitable mildness additive may be prepared as a polymer that comprises hydrophilic monomers, fatty monomers and soft monomers. The monomers are characterized as belonging in various classes by measuring various physical parameters of homopolymers comprised of the corresponding monomer.

Hydrophilic monomers are identified as being soluble in strongly hydrogen bonding solvents or solvent blends that have a solubility parameter of between 9.4 and 14 $(cal/cc)^{1/2.}$ These monomers preferably constitute 5 to 40 mole percent of the mildness additive polymer, and more preferably constitute 10 to 25 mole percent.

Specific examples of hydrophilic monomers to be used in the acrylate polymers of the mildness additives are unesterified $\alpha,\beta$-olefinically unsaturated carboxylic acids such as acrylic acid, methacrylic acid, maleic acid, and itaconic acid. When the monomers are difunctionally acidic, half the amount of this monomer would be used.

Monomers that are fatty in nature comprise acrylates or methacrylates that contain 11 or more carbons in the alcohol portion of the monomer. These monomers preferably constitute 10 to 60 mole percent of the mildness additive polymer, and more preferably constitute 30 to 50 mole percent.

Specific examples of the "fatty" monomers that may be selected for use in the mildness additives of the present invention are:
undecyl acrylate
undecyl methacrylate
lauryl acrylate
lauryl methacrylate
hexadecyl acrylate
hexadecyl methacrylate
octadecyl acrylate
octadecyl methacrylate
stearyl methacrylate

Soft monomers are defined as monomers of which the corresponding homopolymer prepared from the monomer has a glass transition temperature ($T_g$) between -70 and 20°C. These monomers preferably constitute 20 to 70 mole percent of the mildness additive polymer, and more preferably constitute 30 to 50 mole percent.

Especially preferred soft monomers to be used in the acrylate polymers of the mildness additives are:

n-propyl acrylate
n-butyl acrylate
n-butyl methacrylate
iso-butyl acrylate
iso-butyl methacrylate
sec-butyl acrylate
sec-butyl methacrylate
n-amyl acrylate
n-amyl methacrylate
iso-amyl acrylate
iso-amyl methacrylate
n-hexyl acrylate
n-hexyl methacrylate
cyclohexyl acrylate
2-ethylbutyl acrylate
2-ethylbutyl methacrylate
n-heptyl acrylate
n-heptyl methacrylate
n-octyl acrylate
n-octyl methacrylate
2-ethylhexyl acrylate
2-ethylhexyl methacrylate
iso-octyl acrylate
n-nonyl acrylate
n-nonyl methacrylate
n-decyl acrylate
n-decyl methacrylate
iso-decyl acrylate
iso-decyl methacrylate

The incorporation of non-acrylate monomers, such as N-vinyl pyrrolidone, vinyl acetate or of acrylates substituted by functional groups such as amine or hydroxyl functionalities is contemplated, provided that the ultimate mildness additive polymer is soluble in the solvents or solvent blends as indicated. Similarly, "hard" acrylate or methacrylate monomers (monomers wherein the $T_g$ of the corresponding homopolymer is greater than 20° C) may also be incorporated in the mildness additive polymer in a minor amount.

The mildness additives of the present invention are incorporated into conventional cleansing compositions in an amount effective to reduce the drying and irritating effect of these compositions on the skin. Typically, the mildness additive comprises between 0.5 and 5 percent by weight of the total composition.

The mildness additives of the present invention add a substantive component to a cleansing composition due to the physical nature of the polymer and its unique solubility parameter. While not being bound by theory, it is believed that the retention of some of the mildness additive on the skin during and after cleansing leads to simultaneous retention of natural oils and moisture in the skin, so that the skin does not dry out as much as occurs with washing with ordinary cleansing compositions.

Foaming is an important and desirable property of a successful cleansing composition, and because the mildness additives of the present invention act as a poor surfactant, they do not kill the foam provided by the balance of the skin cleansing composition. Additionally, when the skin cleansing composition is in the form of a bar, the mildness additive will reduce the cracking of the bar that would otherwise be observed in use.

The preparation of the polymers from the olefinically unsaturated monomers is well documented in the literature and can be carried out by standard bulk, solution or emulsion techniques. Generally, the latter two are preferred with solution polymerization being most preferred. The polymerization of the monomers is catalyzed by free radical-generating catalysts such as peroxides, and azo catalysts. To be most effective, the reactor for such polymerization should be purged with an inert gas in order to remove traces of oxygen. The solution polymerizations are run in a compatible solvent and the final polymer solution preferably contains 25 to 60 percent solids. Preferably, the polymers are prepared in a fatty acid solvent that is compatible with the ultimate skin cleansing composition, such as Hystrene 1835 (commercially available from Witco Chemical Corp., Memphis, TN) and Emery 627 fatty acids (commercially available from Emery Industries, Inc., Cincinnati, OH).

The molecular weight of the acrylate polymers used in the compositions may vary over a broad range. Preferably, the weight average molecular weight of the polymer is in the range of 50,000 to 2.5 million, and most preferably between 200,000 and 1 million. These weight average molecular weights are measured by GPC based on calibration of polystyrene standards. Generally, these polymers will have a Brookfield viscosity between 50 and 10,000 cps., and preferably between 100 and 1,000 cps., when measured at 10 percent solids at 40 °C.

The mildness additives useful in cleansing compositions are insoluble in water and must be soluble in poorly hydrogen-bonding solvents or solvent blends that have a solubility parameter of between 8.4 and 10.4 $(cal/cc)^{1/2}$. Preferably, the mildness additives are soluble in such solvents or solvent blends that have a solubility parameter of between 8.9 and 9.8 $(cal/cc)^{1/2}$.

The solubility of polymers in solvents with identified solubility parameters is determined according to the method generally described in Polymer Handbook (edited by Bandrup and Immergut, 1966), pages IV-344-358. Specifically, a small amount of solid polymer (about 1 gram) is placed in a test tube and enough of a selected solvent is added such that the final solution has about 3% solids content. The mixture is observed to determine if the polymer is soluble in the solvent. This experiment is repeated over a range of solvents having known solubility parameter values. An extensive list of solvents (classified as either poorly hydrogen-bonding, moderately hydrogen-bonding, or strongly hydrogen-bonding) are described in this handbook.

The soap, soap/synthetic "combo" compositions or the synthetic detergent compositions to which the mildness additives of the present invention are added are the conventional skin cleansing compositions in the art that have the effect of drying or irritating the skin. These compositions typically contain at least 15% of a surfactant by weight. Typically, these compositions contain between 20 and 80% of surfactant by weight. Preferred liquid skin cleansing compositions comprise between 30 and 40% surfactant by weight. Preferred bar skin cleansing compositions comprise between 50 and 80% surfactant by weight.

Traditional soaps comprise the alkali metal salts of long-chain monocarboxylic acids having an alkyl chain length of between about $C_8$ to $C_{22}$, such as sodium tallowate, sodium cocoate, and sodium palmitate. Synthetic detergents are cleansing compositions that contain alkali metal soaps of non-natural acids that are fatty in nature, such as sodium acyl isethionate, sodium lauryl alcohol sulfate, sodium monoalkyl sulfosuccinate, and the like. The soap/synthetic compositions are combinations of soaps and synthetic detergents.

The cleansing compositions of the present invention may additionally comprise pigments, dyes, perfumes, germicides, antioxidants or other such components as are conventional in the cleansing composition art. For example, antibacterial agents such as phenol, cresylic acid, 3,4,4'-trichloro carbanilide and 2-hydroxy-2',4,4'-trichloro diphenyl ether may be incorporated. Where desired, superfatting agents such as unsaponified oil, fatty acid, lanolin, mineral oil, fatty ester or fatty alcohols may also be present. In the case of slightly abrasive soaps, a finely powdered insoluble material such as pumice, alkaline builders such as sodium silicate, sodium carbonate, or trisodium phosphates or microencapsulated moisturizers, such as used in Buf Puf® Daily Cleanser, may be used. Transparent soaps containing the mildness additives of the present invention may be prepared by addition of alcohol, sugar solution and glycerol in a conventional manner. Transparent soaps, it is noted, may also be made by controlled mixing of the soap at specified temperatures in a manner conventional in the art.

The appropriate relative ratios of monomer groups, and consequently the desired solubility parameter of the resulting polymer, are best selected by use of design experiment methods. Thus, a selection of polymers having the indicated solubility parameter is prepared using two or more monomers, with variation of the mole percentage content of the monomers. These mildness additives are incorporated into the selected cleansing compositions, and a test is performed to compare the effect of repeated washing on the skin of hands. One such test is the modified Highley Test described in the Evaluation of Efficacy section below. Additional polymers are prepared that are more closely related to the additive that has the least detrimental or most beneficial effect on skin in the first design experiment. A second series of tests are performed on this second group of compositions to "fine-tune" the selection of monomer content of the mildness additive.

The following examples are illustrative in nature only, and are not intended to limit the scope of the invention.

## PREPARATION OF MILDNESS ADDITIVE

The acrylate polymers were prepared in ethyl acetate and were solvent exchanged at reduced pressures with $C_{18}$ fatty acids (Hystrene 1835, commercially available from Witco Chemical Corp., Memphis, TN), to provide a polymer in Hystrene 1835 content of 10% solids. These mixtures were added

to about one third the amount by weight of a 1:1 ratio Tallow/coco fatty acid blend for incorporation into the bars. Some of the polymers prepared and evaluated are shown in Table I.

PREPARATION OF TRANSPARENT COMBO-BAR

| Solution A: | | % by wt. |
|---|---|---|
| 1. | Triethanolamine, 99% | 27.5 |
| 2. | sorbitol, 70% | 3.93 |
| 3. | glycerine, 99% | 3.53 |
| 4. | "Surfine AZI-A"* | 2.50 |
| 5. | "Finsolve TN"* | 2.00 |
| 6. | "Tauranol I-78"* | 10.00 |

"Surfine AZI-A", "Finsolve TN" and "Tauranol I-78" are all commercially available from Finetex Inc., Elmwood Park, NJ.

Liquids 1-5 were combined and heated with stirring under $N_2$ to 70 °C ± 5 °C. Tauranol (6) was added, and the temperature of the composition was maintained until it dissolved.

| Solution B: | | % by wt. |
|---|---|---|
| 7. | 10% polymer in Hystrene 1835 | 26.55 |
| 8. | Tallow/coco fatty acid blend (1:1 ratio) | 8.50 |

Components 7 and 8 were combined and heated to 60 °C ± 2 °C until they dissolved.

| Solution C: | | % by wt. |
|---|---|---|
| 9. | NaOH | 4.04 |
| 10. | NaCl | 0.21 |
| 11. | Water | 11.24 |

Components 9 and 10 were combined in 11.

Solution B (at 60 °C ± 2 °C) and solution C were added simultaneously to solution A at 70 °C ± 5 °C with good stirring and $N_2$ purge for 10 minutes. The mixer and $N_2$ gas were turned off and the bubbles were allowed to rise. The foam was skimmed off and the liquid was poured into bar soap molds.

Evaluation of Efficacy

The mildness of the bar soaps was evaluated via a modified Highley induced dryness test which measures the effectiveness of a product to prevent the formation of dry, chapped hands. In this test, participants wash the backs of one of their hands with a test bar and the other with a control bar not containing the polymer. The hands are air dried after each washing. This is repeated for a total of 5 washings each day for four days. One hour after the last washing each day the hands are scored on a scale of 0-5 with respect to dry, scaly chapped hands. A 0 rating is best and a 5 rating worst. The Highley values appear in Table I, which are normalized with the control so that a bar with no additive would receive a Highley score of 0 and higher numbers indicate better prevention of dry, chapped hands.

**TABLE I**

**Polymer Composition and Highley Scores**

A. First Design

Polymer Mole Percent

| | Acrylic Acid | Stearyl Methacrylate | Iso-octyl Acrylate | Highley Score |
|---|---|---|---|---|
| 1. | 15 | 20 | 65 | 1.17 |
| 2. | 35 | 20 | 45 | 0.48 |
| 3. | 15 | 40 | 45 | 2.66 |
| 4. | 35 | 40 | 25 | 0.75 |
| 5. | 25 | 30 | 45 | 0.52 |
| 6. | 25 | 30 | 45 | 0.28 |

B. Second Design

| | | | | |
|---|---|---|---|---|
| 7. | 10 | 35 | 55 | 0.56 |
| 8. | 20 | 35 | 45 | 0.21 |
| 9. | 10 | 45 | 45 | 0.29 |
| 10. | 20 | 45 | 35 | 1.02 |
| 11. | 15 | 40 | 45 | 0.44 |
| 12. | 15 | 40 | 45 | 0.75 |

Note: The Highley scores of the First and Second Design cannot be compared to each other. The First Design experiment was conducted with no pretreatment of the hands. In the Second Design experiment, the subjects were first required to wash their hands with a harsh soap, so the Highley score actually represents an improvement over initially dry skin.

A score of 2.66 for No. 3 polymer represents a very, very significant improvement over the control bar for the prevention of dry skin. The second design experiment shows that No. 10 polymer is an even better mildness additive for this particular cleansing formulation.

## Claims

**1.** A skin-cleansing composition consisting essentially of

a) a cleansing component selected from a soap composition, a soap/synthetic "combo" composition or a synthetic detergent composition, said cleansing component characterized by having the effect of drying or irritating skin, and

b) a non-water soluble acrylate polymer that is soluble in poorly hydrogen bonding solvents or solvent blends that have a solubility parameter of between 8.4 and 10.4 $(cal/cc)^{1/2}$ in an amount effective to reduce the drying or irritating effect of component a) on the skin, said polymer

comprising monomers of the formula:

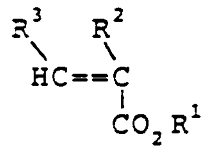

wherein $R^1$ is H or an alkyl radical containing 1 to 18 carbon atoms in cyclic, straight- or branched-chain configuration and is saturated or unsaturated; $R^2$ is hydrogen, lower alkyl or $-CH_2CO_2R^1$; and $R^3$ is hydrogen, methyl or $--CO_2R^1$; provided, that when $R^2$ is not hydrogen, $R^3$ is hydrogen, and when $R^3$ is not hydrogen, $R^2$ is hydrogen.

**2.** The composition of claim 1, wherein said polymer comprises
a) 5 to 40 mole percent of an monomer of which the corresponding homopolymer is hydrophilic;
b) 10 to 60 mole percent of an monomer of which the corresponding homopolymer is fatty in nature; and
c) 20 to 70 mole percent of an monomer of which the corresponding homopolymer is soft in nature.

**3.** The composition of claim 2, wherein said polymer comprises
10-25 mole percent of monomer a);
30-50 mole percent of monomer b); and
30-50 mole percent of monomer c).

**4.** The composition of claim 2, wherein said polymer comprises
a) 5 to 40 mole percent of a hydrophilic monomer selected from acrylic acid, methacrylic acid, maleic acid and itaconic acid;
b) 10 to 60 mole percent of a fatty monomer selected from undecyl acrylate, undecyl methacrylate, lauryl acrylate, lauryl methacrylate, hexadecyl acrylate, hexadecyl methacrylate, octadecyl acrylate, octadecyl methacrylate and stearyl methacrylate; and
c) 20 to 70 mole percent of a soft monomer selected from n-propyl acrylate, n-butyl acrylate, n-butyl methacrylate, iso-butyl acrylate, iso-butyl methacrylate, sec-butyl acrylate, sec-butyl methacrylate, n-amyl acrylate, n-amyl methacrylate, iso-amyl acrylate, iso-amyl methacrylate, n-hexyl acrylate, n-hexyl methacrylate, cyclohexyl acrylate, 2-ethylbutyl acrylate, 2-ethylbutyl methacrylate, n-heptyl acrylate, n-heptyl methacrylate, n-octyl acrylate, n-octyl methacrylate, 2-ethylhexyl acrylate, 2-ethylhexyl methacrylate, iso-octyl acrylate, n-nonyl acrylate, n-nonyl methacrylate, n-decyl acrylate, n-decyl methacrylate, iso-decyl acrylate and iso-decyl methacrylate.

**5.** The composition of claim 4, wherein said polymer comprises
a) 10 to 25 mole percent of a hydrophilic monomer selected from acrylic acid, methacrylic acid, maleic acid and itaconic acid;
b) 30 to 50 mole percent of a fatty monomer selected from undecyl acrylate, undecyl methacrylate, lauryl acrylate, lauryl methacrylate, hexadecyl acrylate, hexadecyl methacrylate, octadecyl acrylate, octadecyl methacrylate and stearyl methacrylate; and
c) 30 to 50 mole percent of a soft monomer selected from n-propyl acrylate, n-butyl acrylate, n-butyl methacrylate, iso-butyl acrylate, iso-butyl methacrylate, sec-butyl acrylate, sec-butyl methacrylate, n-amyl acrylate, n-amyl methacrylate, iso-amyl acrylate, iso-amyl methacrylate, n-hexyl acrylate, n-hexyl methacrylate, cyclohexyl acrylate, 2-ethylbutyl acrylate, 2-ethylbutyl methacrylate, n-heptyl acrylate, n-heptyl methacrylate n-octyl acrylate, n-octyl methacrylate, 2-ethylhexyl acrylate, 2-ethylhexyl methacrylate, iso-octyl acrylate, n-nonyl acrylate, n-nonyl methacrylate, n-decyl acrylate, n-decyl methacrylate, iso-decyl acrylate and iso-decyl methacrylate.

**6.** The composition of claim 5, wherein said polymer comprises
a) 10 to 25 mole percent of a hydrophilic monomer selected from acrylic acid and methacrylic acid;
b) 30 to 50 mole percent of a fatty monomer that is stearyl methacrylate; and

c) 30 to 50 mole percent of a soft monomer selected from n-butyl acrylate, n-butyl methacrylate, iso-butyl acrylate, iso-butyl methacrylate, sec-butyl acrylate, sec-butyl methacrylate, n-amyl acrylate, n-amyl methacrylate, iso-amyl acrylate, iso-amyl methacrylate, n-hexyl acrylate, n-hexyl methacrylate, cyclohexyl acrylate, 2-ethylbutyl acrylate, 2-ethylbutyl methacrylate, n-heptyl acrylate, n-heptyl methacrylate, n-octyl acrylate, n-octyl methacrylate, 2-ethylhexyl acrylate, 2-ethylhexyl methacrylate and iso-octyl acrylate.

**7.** The composition of any preceding claim, wherein said polymer comprises between 0.5 and 5 percent by weight of the total composition.

**8.** The composition of any preceding claim comprising an alkali metal soap of a fatty acid having an alkyl chain length of between $C_8$ to $C_{22}$.

**9.** The composition of any preceding claim further comprising glycerine.

**10.** The composition of any preceding claim wherein said composition is in the form of a bar.

**11.** The composition of any one of preceding claims 1 to 9 wherein said composition is in the form of a liquid.

**12.** The composition of any preceding claim wherein said polymer is soluble in poorly hydrogen bonding solvents or solvent blends that have a solubility parameter of between 8.9 and 9.8 $(cal/cc)^{1/2}$.

**13.** The composition of any preceding claim wherein said cleansing component comprises at least 15% by weight surfactant.

**14.** The composition of claim 13 wherein said cleansing component comprises between 20 and 80% by weight surfactant.

**15.** The composition of claim 13 wherein said cleansing component comprises between 50 and 80% by weight surfactant.

**16.** The composition of claim 13 wherein said cleansing component comprises between 30 and 40% by weight surfactant.

### Patentansprüche

**1.** Hautreinigungsmittel, im wesentlichen bestehend aus:

a) einer reinigenden Komponente, ausgewählt aus einer Seifenzusammensetzung, einer Seife/synthetischen "Combo"-Zusammensetzung oder einem synthetischen Detergens, welche reinigende Komponente dadurch ausgezeichnet ist, daß sie den Effekt des Trocknens oder der Irritation der Haut hat, und

b) einem nichtwasserlöslichen Acrylatpolymer, welches in schwach wasserstoffbindenden Lösemitteln oder Lösemittelgemischen löslich ist, die einen Löslichkeitsparameter zwischen 8,4 und 10,4 $(cal/cm^3)^{1/2}$ aufweisen, und in einer wirksamen Menge, um den Effekt des Trocknens oder der Irritation von Komponente a) auf der Haut herabzusetzen, welches Polymer Monomere der Formel umfaßt:

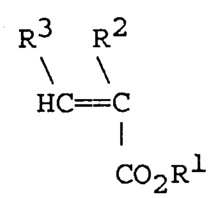

worin sind: $R^1$ Wasserstoff oder ein Alkylradikal mit 1 bis 18 Kohlenstoffatomen in zyklischer, geradkettiger oder verzweigtkettiger Konfiguration und das gesättigt oder ungesättigt ist; $R^2$ Wasser-

stoff, niederes Alkyl oder -$CH_2CO_2R^1$; und $R^3$ Wasserstoff, Methyl oder -$CO_2R^1$, unter der Voraussetzung, daß, wenn $R^2$ nicht Wasserstoff ist, $R^3$ Wasserstoff ist, und, wenn $R^3$ nicht Wasserstoff ist, $R^2$ Wasserstoff ist.

**2.** Zusammensetzung nach Anspruch 1, bei welcher das Polymer aufweist:
a) 5 bis 40 Molprozent eines Monomers, von dem das entsprechende Homopolymer hydrophil ist,
b) 10 bis 60 Molprozent eines Monomers, von dem das entsprechende Homopolymer von Natur aus fettig ist; und
c) 20 bis 70 Molprozent eines Monomers, von dem das entsprechende Homopolymer von Natur aus weich ist.

**3.** Zusammensetzung nach Anspruch 2, bei welcher das Polymer aufweist:
10 bis 25 Molprozent Monomer a),
30 bis 50 Molprozent Monomer b) und
30 bis 50 Molprozent Monomer c).

**4.** Zusammensetzung nach Anspruch 2, bei welcher das Polymer aufweist:
a) 5 bis 40 Molprozent eines hydrophilen Monomers, ausgewählt aus: Arylsäure, Methacrylsäure, Maleinsäure und Itaconsäure;
b) 10 bis 60 Molprozent eines aliphatischen Monomers, ausgewählt aus: Undecylacrylat, Undecylmethacrylat, Laurylacrylat, Laurylmethacrylat, Hexadecylacrylat, Hexadecylmethacrylat, Octadecylacrylat, Octadecylmethacrylat und Stearylmethacrylat sowie
c) 20 bis 70 Molprozent eines weichen Monomers, ausgewählt aus: n-Propylacrylat, n-Butylacrylat, n-Butylmethacrylat, Isobutylacrylat, Isbutylmethacrylat, *sec*-Butylacrylat, *sec*-Butylmethacrylat, n-Amylacrylat, n-Amylmethacrylat, Isoamylacrylat, Isoamylmethacrylat, n-Hexlacrylat, n-Hexylmethacrylat, Cyclohexylacrylat, 2-Ethylbutylacrylat, 2-Ethylbutylmethacrylat, n-Heptylacrylat, n-Heptylmethacrylat, n-Octylacrylat, n-Octylmethacrylat, 2-Ethylhexylacrylat, 2-Ethylhexylmethacrylat, Isooctylacrylat, n-Nonylacrylat, n-Nonylmethacrylat, n-Decylacrylat, n-Decylmethacrylat, Isodecylacrylat und Isodecylmethacrylat.

**5.** Zusammensetzung nach Anspruch 4, bei welcher das Polymer aufweist:
a) 10 bis 25 Molprozent eines hydrophilen Monomers, ausgewählt aus: Acrylsäure, Methacrylsäure, Maleinsäure und Itaconsäure;
b) 30 bis 50 Molprozent eines aliphatischen Monomers, ausgewählt aus: Undecylacrylat, Undecylmethacrylat, Laurylacrylat, Laurylmethacrylat, Hexadecylacrylat, Hexadecylmethacrylat, Octadecylacrylat, Octadecylmethacrylat und Stearylmethacrylat; sowie
c) 30 bis 50 Molprozent eines weichen Monomers, ausgewählt aus: n-Propylacrylat, n-Butylacrylat, n-Butylmethacrylat, Isobutylacrylat, Isobutylmethacrylat, *sec*-Butylacrylat, *sec*-Butylmethacrylat, n-Amylacrylat, n-Amylmethacrylat, Isoamylacrylat, Isoamylmethacrylat, n-Hexylacrylat, n-Hexylmethacrylat, Cyclohexylacrylat, 2-Ethylbutylacrylat, 2-Ethylbutylmethacrylat, n-Heptylacrylat, n-Heptylmethacrylat, n-Octylacrylat, n-Octylmethacrylat, 2-Ethylhexylacrylat, 2-Ethylhexylmethacrylat, Isooctylacrylat, n-Nonylacrylat, n-Nonylmethacrylat, n-Decylacrylat, n-Decylmethacrylat, Isodecylacrylat und Isodecylmethacrylat.

**6.** Zusammensetzung nach Anspruch 5, bei welcher das Polymer aufweist:
a) 10 bis 25 Molprozent eines hydrophilen Monomers, ausgewählt aus: Acrylsäure und Methacrylsäure;
b) 30 bis 50 Molprozent eines aliphatischen Monomers, welches Stearylmethacrylat ist; und
c) 30 bis 50 Molprozent eines weichen Monomers, ausgewählt aus: n-Butylacrylat, n-Butylmethacrylat, Isobutylacrylat, Isobutylmethacrylat, *sec*-Butylacrylat, *sec*-Butylmethacrylat, n-Amylacrylat, n-Amylmethacrylat, Isoamylacrylat, Isoamylmethacrylat, n-Hexylacrylat, n-Hexylmethacrylat, Cyclohexylacrylat, 2-Ethylbutylacrylat, 2-Ethylbutylmethacrylat, n-Heptylacrylat, n-Heptylmethacrylat, n-Octylacrylat, n-Octylmethacrylat, 2-Ethylhexylacrylat, 2-Ethylhexylmethacrylat und Isooctylacrylat.

**7.** Zusammensetzung nach einem der vorgenannten Ansprüche, bei welcher das Polymer zwischen 0,5 und 5 Gewichtsprozent der Gesamtzusammensetzung aufweist.

**8.** Zusammensetzung nach einem der vorgenannten Ansprüche, umfassend eine Alkimetallseife einer Fettsäure mit einer Alkylkettenlänge zwischen $C_8$ bis $C_{22}$.

**9.** Zusammensetzung nach einem der vorgenannten Ansprüche, ferner umfassend Glyzerin.

**10.** Zusammensetzung nach einem der vorgenannten Ansprüche, bei welcher die Zusammensetzung in Form eines Stückes vorliegt.

**11.** Zusammensetzung nach einem der Ansprüche 1 bis 9, bei welcher die Zusammensetzung in Form einer Flüssigkeit vorliegt.

**12.** Zusammensetzung nach einem der vorgenannten Ansprüche, bei welcher das Polymer in schwach wasserstoffbindenden Lösemitteln oder Lösemittelgemischen löslich ist, die einen Löslichkeitsparameter zwischen 8,9 und 9,8 $(cal/cm^3)^{1/2}$ haben.

**13.** Zusammensetzung nach einem der vorgenannten Ansprüche, bei welcher die reinigende Komponente mindestens 15 Gewichtsprozent Tensid umfaßt.

**14.** Zusammensetzung nach Anspruch 13, bei welcher die reinigende Komponente zwischen 20 % und 80 Gewichtsprozent Tensid umfaßt.

**15.** Zusammensetzung nach Anspruch 13, bei welcher die reinigende Komponente zwischen 50 % und 80 Gewichtsprozent Tensid umfaßt.

**16.** Zusammensetzung nach Anspruch 13, bei welcher die reinigende Komponente zwischen 30 % und 40 Gewichtsprozent Tensid umfaßt.

**Revendications**

**1.** Composition nettoyante pour la peau, constituée essentiellement :

a) d'un constituant nettoyant, choisi parmi les compositions de savon, les compositions "combinées" savon/synthétique ou les compositions détergentes synthétiques, le constituant nettoyant étant caractérisé en ce qu'il a pour effet de sécher ou d'irriter la peau, et

b) d'un polymère d'un acrylate, non soluble dans l'eau, qui est soluble dans les solvants à faible liaison hydrogène ou les mélanges de solvants ayant un paramètre de solubilité de 8,4 et 10,4 $(cal/cm^3)^{1/2}$, en une quantité suffisante pour réduire l'effet de séchage ou d'irritation du constituant a) sur la peau, ce polymère comprenant des monomères de formule :

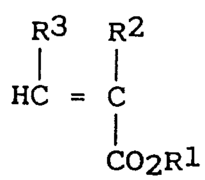

dans laquelle $R^1$ est H ou un radical alkyle ayant de 1 à 18 atomes de carbone selon une configuration cyclique ou à chaîne droite ou ramifiée, et est saturé ou insaturé ; $R^2$ est un hydrogène ou un radical alkyle inférieur ou $-CH_2CO_2R^1$ ; et $R^3$ est un hydrogène ou un radical méthyle ou $-CO_2R^1$ ; du moment que, quand $R^2$ n'est pas un hydrogène, $R^3$ est un hydrogène, et que, quand $R^3$ n'est pas un hydrogène, $R^2$ est un hydrogène.

**2.** Composition selon la revendication 1, dans laquelle le polymère comprend :

a) de 5 à 40 % en moles d'un monomère dont l'homopolymère correspondant est hydrophile ;

b) de 10 à 60 % en moles d'un monomère dont l'homopolymère correspondant est de nature grasse ; et

c) de 20 à 70 % en moles d'un monomère dont l'homopolymère correspondant est mou.

**3.** Composition selon la revendication 2, dans laquelle le monomère comprend :
de 10 à 25 % en moles du monomère a) ;
de 30 à 50 % en moles du monomère b) ; et
de 30 à 50 % en moles du monomère c).

**4.** Composition selon la revendication 2, dans laquelle le polymère comprend :
a) de 5 à 40 % en moles d'un monomère hydrophile choisi parmi l'ensemble comprenant l'acide acrylique, l'acide méthacrylique, l'acide maléique et l'acide itaconique ;
b) de 10 à 60 % en modes d'un monomère gras choisi parmi l'ensemble comprenant l'acrylate d'undécyle, le méthacrylate d'undécyle, l'acrylate de lauryle, le méthacrylate de lauryle, l'acrylate d'hexadécyle, le méthacrylate d'hexadécyle, l'acrylate d'octadécyle, le méthacrylate d'octadécyle et le méthacrylate de stéaryle ; et
c) de 20 à 70 % en modes d'un monomère mou choisi parmi l'ensemble comprenant d'acrylate de n-propyle, l'acrylate de n-butyle, le méthacrylate de n-butyle, l'acrylate de d'isobutyle, le méthacry-late d'isobutyle, l'acrylate de sec-butyle, le méthacrylate de sec-butyle, l'acrylate de n-amyle, le méthacrylate de n-amyle, l'acrylate de d'isoamyle, le méthacrylate d'isoamyle, l'acrylate de n-hexyle, le méthacrylate de n-hexyle, l'acrylate de cyclohexyle, l'acrylate de 2-éthylbutyle, le méthacrylate de 2-éthylbutyle, l'acrylate de n-heptyle, le méthacrylate de n-heptyle, l'acrylate de n-octyle, le méthacrylate de n-octyle, l'acrylate de 2-éthylhexyle, le méthacrylate de 2-éthylhexyle, l'acry-late d'isooctyle, l'acrylate de n-nonyle, de méthacry-late de n-nonyle, l'acrylate de n-décyle, le méthacry-late de n-décyle, l'acrylate d'isodécyle et le méthacry-late d'isodécyle.

**5.** Composition selon la revendication 4, dans laquelle le polymère comprend :
a) de 10 à 25 % en moles d'un monomère hydrophile choisi parmi l'ensemble comprenant l'acide acrylique, l'acide méthacrylique, l'acide maléique et l'acide itaconique ;
b) de 30 à 50 % en modes d'un monomère gras choisi parmi l'ensemble comprenant l'acrylate d'undécyle, le méthacrylate d'undécyle, l'acrylate de lauryle, le méthacrylate de lauryle, l'acrylate d'hexadécyle, le méthacrylate d'hexadécyle, l'acrylate d'octadécyle, le méthacrylate d'octadécyle et le méthacrylate de stéaryle ; et
c) de 30 à 50 % en modes d'un monomère mou choisi parmi l'ensemble comprenant l'acrylate de n-propyle, l'acrylate de n-butyle, le méthacrylate de n-butyle, l'acrylate de d'isobutyle, le méthacry-late d'isobutyle, l'acrylate de sec-butyle, le méthacrylate de sec-butyle, l'acrylate de n-amyle, le méthacrylate de n-amyle, l'acrylate de d'isoamyle, le méthacrylate d'isoamyle, l'acrylate de n-hexyle, le méthacrylate de n-hexyle, l'acrylate de cyclohexyle, l'acrylate de 2-éthylbutyle, le méthacrylate de 2-éthylbutyle, l'acrylate de n-heptyle, le méthacrylate de n-heptyle, l'acrylate de n-octyle, le méthacrylate de n-octyle, l'acrylate de 2-éthylhexyle, le méthacrylate de 2-éthylhexyle, l'acrylate d'isooctyle, l'acrylate de n-nonyle, le méthacrylate de n-nonyle, l'acrylate de n-décyle, le méthacrylate de n-décyle, l'acrylate d'isodécyle et le méthacrylate d'isodécyle.

**6.** Composition selon la revendication 5, dans laquelle le polymère comprend :
a) de 10 à 25 % en modes d'un monomère hydrophile choisi parmi l'ensemble comprenant l'acide acrylique et l'acide méthacrylique ;
b) de 30 à 50 % en modes d'un monomère gras qui est le méthacrylate de stéaryle ; et
c) de 30 à 50 % en modes d'un monomère mou choisi parmi l'ensemble comprenant l'acrylate de n-butyle, le méthacrylate de n-butyle, l'acrylate d'isobutyle, le méthacrylate d'isobutyle, l'acrylate de sec-butyle, le méthacrylate de sec-butyle, l'acrylate de n-amyle, le méthacrylate de n-amyle, l'acrylate d'isoamyle, le méthacrylate d'isoamyle, l'acrylate de n-hexyle, le méthacrylate de n-hexyle, l'acrylate de cyclohexyle, l'acrylate de 2-éthylbutyle, le méthacrylate de 2-éthylbutyle, l'acrylate de n-heptyle, le méthacrylate de n-heptyle, l'acrylate de n-octyle, le méthacrylate de n-octyle, l'acrylate de 2-éthylhexyle, le méthacrylate de 2-éthylhexyle, et l'acrylate d'isooctyle.

**7.** Composition selon d'une quelconque des revendications précédentes, dans laquelle le polymère compte pour 0,5 à 5 % en poids de la composition totale.

**8.** Composition selon d'une quelconque des revendications précédentes, comprenant un sel d'un métal alcalin d'un acide gras ayant une longueur de chaîne alkyle de $C_8$ à $C_{22}$.

9. Composition selon l'une quelconque des revendications précédentes, qui comprend en outre du glycérol.

10. Composition selon l'une quelconque des revendications précédentes, dans laquelle la composition se présente sous forme d'une barre.

11. Composition selon l'une quelconque des revendications précédentes 1 à 9, dans laquelle la composition se présente sous forme d'un liquide.

12. Composition selon l'une quelconque des revendications précédentes, dans laquelle de polymère est soluble dans des solvants à faible liaison hydrogène ou des mélanges de solvants ayant un paramètre de solubilité compris entre 8,9 et 9,8 $(cal/cm^3)^{1/2}$.

13. Composition selon d'une quelconque des revendications précédentes, dans laquelle de constituant nettoyant comprend au moins 15 % en poids d'un tensioactif.

14. Composition selon la revendication 13, dans laquelle le constituant nettoyant comprend de 20 à 80 % en poids d'un tensioactif.

15. Composition selon la revendication 13, dans laquelle de constituant nettoyant comprend de 50 à 80 % en poids de tensioactif.

16. Composition selon la revendication 13, dans laquelle le constituant nettoyant comprend de 30 à 40 % en poids de tensioactif.